# DEMANDE DE BREVET EUROPEEN

(11) **EP 0 875 242 A2**
(43) Date de publication de la demande: **04.11.1998**
(21) Numéro de dépôt: 98400744.3
(22) Date de dépôt: 30.03.1998
(51) Int. Cl.: A61K 7/48, A61K 7/027, C08L 27/12

(54) **Composition comprenant un polymère fluoré en dispersion**

(30) Priorité: 28.04.1997 FR 9705441
(71) Demandeur: L'OREAL, 75008 Paris (FR)
(72) Inventeur: De la Poterie, Valérie, 77820 Le Chatelet en Brie (FR)
(74) Mandataire: Lhoste, Catherine

(57) **Abrégé**

La présente demande se rapporte à une composition susceptible d'être appliquée sur la peau, les semi-muqueuses et/ou les muqueuses d'être humain, comprenant un système polymérique qui comprend une dispersion aqueuse de particules de polymère fluoré filmogène.

La composition permet d'obtenir un film de très bonne tenue, qui ne transfère pas, et ne migre pas au cours du temps. Elle peut se présenter sous la forme d'un produit de maquillage, notamment en tant que rouge à lèvres, fond de teint, fard à joues, fard à paupières, eye-liner, produit de maquillage du corps ; d'une composition de soin, notamment soin des lèvres ; d'une composition solaire ou auto-bronzante ; d'une composition dermatologique ou pharmaceutique.

L'invention a également pour objet l'utilisation d'un tel système polymérique pour obtenir un film capable de suivre le mouvement du support.

## Description

La présente invention a trait à une composition notamment cosmétique susceptible d'être appliquée sur la peau, les semi-muqueuses et/ou les muqueuses d'être humain. Cette composition comprend en particulier une dispersion aqueuse de particules de polymère filmogène et peut être utilisée en tant que produit de maquillage, aussi bien du corps que du visage et des fibres kératiniques.

Les compositions à appliquer sur la peau, les semi-muqueuses et/ou les muqueuses, de type rouge à lèvres ou fond de teint, se présentent généralement sous forme de stick, de pâte souple ou de pâte coulée, et comprennent des corps gras tels que des huiles, des composés pâteux et/ou des cires, et une phase particulaire généralement composée de charges et de pigments. Ces compositions, lorsqu'elles sont appliquées sur la peau, présentent toutefois l'inconvénient de transférer. On entend par là le fait que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau, les semi-muqueuses ou les muqueuses, et la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, produites par les mouvements des paupières. Tous ces phénomènes engendrent un effet inesthétique que l'on souhaite bien évidemment éviter.

On connaît des compositions de maquillage dites 'sans transfert', susceptibles de pallier les inconvénients ci-dessus mentionnés ; elles comprennent généralement, parmi les corps gras qui les composent, des huiles volatiles, notamment des huiles de silicone volatiles et/ou des huiles hydrocarbonées volatiles. Toutefois, ces compositions conduisent à un maquillage très mat.

Ainsi, il a été envisagé des compositions liquide de rouge à lèvres 'sans transfert' contenant de 1 à 70% en poids de résine de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).

On connaît également des rouges à lèvres'sans transfert' contenant une silicone volatile et une résine de silicone comportant une chaîne estérifiée pendante ayant au moins 12 atomes de carbone. Le film de rouge à lèvres présente notamment l'inconvénient de manquer de confort à l'application, en particulier d'être trop sec. Ainsi, d'une manière générale, l'association d'huiles volatiles avec certains composés siliconés permet d'obtenir un résultat 'sans transfert' satisfaisant. Toutefois, les films obtenus après application de ces compositions et évaporation des volatils présentent néanmoins l'inconvénient d'être relativement mats, et conduisent ainsi à un maquillage peu brillant, et peu confortable.

Il subsiste donc le besoin d'une composition cosmétique qui transfère peu ou pas du tout, c'est-à-dire une composition 'sans transfert', tout en possédant de bonnes propriétés cosmétiques, et en particulier permettant l'obtention d'un film qui peut être, au choix, plus ou moins brillant, et confortable.

La présente invention a pour but de proposer une telle composition, qui permet d'obtenir un film de très bonne tenue, qui ne transfère pas et ne tache pas un support avec lequel il serait en contact, et qui ne migre pas au cours du temps, tout en permettant d'obtenir un maquillage et/ou un film brillant et/ou confortable.

Ainsi, un objet de l'invention est une composition susceptible d'être appliquée sur la peau, les semi-muqueuses et/ou les muqueuses d'être humain, comprenant un système polymérique qui comprend une dispersion aqueuse de particules de polymère fluoré filmogène. Notamment, le système polymérique est présent en une quantité suffisante pour permettre l'obtention d'un film capable de suivre le mouvement de la peau, des semi-muqueuses et/ou des muqueuses d'être humain sur lequel il est appliqué. Le système polymérique est en particulier présent en une quantité suffisante pour permettre l'obtention d'un film sans transfert et/ou de bonne adhérence sur le support. Par ailleurs, le système polymérique est de préférence plus présent en une quantité suffisante pour permettre l'obtention d'un film brillant et/ou confortable.

L'utilisation d'une dispersion de particules de polymère permet l'obtention d'un vrai film isolable, adhérent bien sur la peau, muqueuses et/ou semi-muqueuses d'être humain, non glissant, non gras et de bonnes propriétés mécaniques.

L'invention a également pour objet une composition de rouge à lèvres ou de soin des lèvres, caractérisée par le fait que la composition est conforme à celle décrite ci-dessus. Elle a aussi pour objet une compositions de maquillage du corps, notamment du type semi-permanent (tatouage).

L'invention a encore pour objet un procédé de maquillage et un procédé de traitement non thérapeutique, d'un support choisi parmi la peau, les semi-muqueuses et les muqueuses d'être humain, caractérisé par le fait que l'on applique sur ledit support, une composition telle que décrite ci-dessus.

L'invention a également pour objet l'utilisation d'un système polymérique comprenant une dispersion aqueuse de particules de polymère fluoré filmogène, dans une composition à appliquer sur un support choisi parmi la peau, les semi-muqueuses et les muqueuses d'être humain, pour obtenir un film capable de suivre le mouvement dudit support.

On a constaté que la composition selon l'invention est facilement applicable et s'étale aisément et uniformément sur la peau, les semi-muqueuses et les muqueuses d'être humain, en particulier sur les lèvres du visage.

La composition selon l'invention trouve notamment une application particulièrement intéressante dans le domaine du soin et/ou du maquillage de la peau, des muqueuses et/ou des semi-muqueuses. On entend notamment par muqueuse, la partie interne de la paupière inférieure ; parmi les semi-muqueuses, on entend plus particulièrement les lèvres du visage.

La composition selon l'invention permet l'obtention d'un film homogène, qui présente une texture légère et reste confortable à porter tout au long de la journée. Le film n'est pas du tout collant, tout en étant mou, souple, élastique et flexible sur la peau ; il suit les mouvements du support sur lequel il est déposé. Il adhère notamment parfaitement bien sur les lèvres du visage. Le film est de très bonne tenue et reste adhérent au cours du temps ; il ne transfère pas, ne migre pas, ne tache pas.

Cette composition présente en particulier une bonne résistance à l'eau et une bonne tenue dans le temps tout en conservant de bonnes propriétés cosmétiques, et une grande aptitude à former des films homogènes et continus. D'autre part, le film obtenu peut être très brillant, ou plus ou moins mat, selon la nature des constituants de la composition, d'où une gamme plus étendue de produits de maquillage, brillants ou mats, au choix.

La composition selon l'invention trouve une application toute particulière dans le domaine des produits de maquillage des lèvres du visage, notamment en tant que rouge à lèvres ou 'laque à lèvres' mais aussi de maquillage du corps.

La composition selon l'invention comprend donc un système polymérique qui comprend au moins une dispersion aqueuse d'au moins un polymère fluoré filmogène, les particules étant notamment dispersées dans un milieu cosmétiquement ou dermatologiquement acceptable. De préférence, on utilise une composition à phase continue aqueuse.

Le système polymérique doit être présent en quantité suffisante et de nature à permettre l'obtention d'un film capable de suivre le mouvements de la peau, des semi-muqueuses et/ou des muqueuses sur lequel il est appliqué, notamment le mouvement des lèvres du visage. Il peut également être présent en une quantité suffisante pour permettre l'obtention d'un film sans transfert et/ou l'obtention d'un film brillant.

Parmi les polymères fluorés filmogènes utilisables dans la présente invention, on peut citer plus particulièrement les polymères radicalaires comprenant au moins un groupement fluoré. Les polymères sont en particulier des perfluoroalkyl(méth)acrylates.

Ces polymères fluorés sont des polymères à squelette hydrocarboné dont au moins une partie des hydrogènes est substituée par des atomes de fluor. Ces polymères, autrement dit, ne comportent pas de groupement siliconés. Ils sont parfaitement compatibles avec tous les produits hydrocarbonés comme les huiles et cires classiquement utilisées en cosmétique, notamment pour leur côté confort et hydratant.

Par polymère radicalaire, on entend un polymère obtenu par polymérisation de monomères à insaturation notamment éthylénique, chaque monomère étant susceptible de s'homopolymériser. Les polymères de type radicalaires peuvent être notamment des polymères ou des copolymères vinyliques, notamment des polymères acryliques. Les polymères vinyliques peuvent résulter de la polymérisation de monomères à insaturation éthylénique ayant au moins un groupement acide et/ou des esters de ces monomères acides et/ou des amides de ces monomères acides.

Parmi les polymères fluorés utilisables dans l'invention, on peut citer les copolymères fluorés résultant de la copolymérisation d'au moins un premier monomère fluoré vinylique et d'au moins un second monomère de formule (A) : 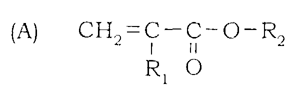 dans laquelle :
- R1 représente un atome d'hydrogène ou un radical alkyle en C1-C4 et
- R2 représente un radical alkyle en C1-C20, de préférence C2-C8, un radical hydroxycarboné en C2-C6, de préférence C2-C4, ou un radical -(CH₂)ₙ-NH-R3 avec R3 représentant un alkyle en C1-C6 ou un cycloalkyle et n étant un entier allant de 1 à 4.

Par "fluoré", on entend dans la présente description une substitution totale ou partielle des atomes d'hydrogène de la chaîne alkyle par des atomes de fluor. De préférence, la substitution est totale. Par "radical hydroxycarboné", il faut comprendre un radical hydroxyalkyle ou hydroxyalkylène.

Selon l'invention, les radicaux alkyles, fluorés ou non, et les radicaux alkylénés peuvent être linéaires ou ramifiés.

De préférence, on choisit le radical R1 du monomère de formule A parmi l'hydrogène ou un radical méthyle, éthyle, n-butyle ou isopropyle. En particulier, on choisit les monomères de formule A parmi l'acide acrylique, l'acide méthacrylique, le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle, le (méth)acrylate d'hydroxyéthyle, le (méth)acrylate de 2-hydroxypropyle, leurs mélanges.

De façon avantageuse, le premier monomère fluoré est un monomère de type acrylique présentant en particulier la formule (B) suivante : 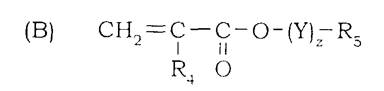 dans laquelle :
- R4 représente un atome d'hydrogène ou un radical alkyle en C1-C4,
- Y représente un radical alkylène en C1-C6 et
- z est égal à 0 ou 1,
- R5 représente un radical alkyle en C1-C20 dont tout ou partie des atomes d'hydrogène est remplacé par des atomes de fluor.

A titre d'exemple, le premier monomère fluoré peut être de formule : 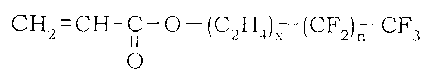 avec n allant de 4 à 16, de préférence égal à 5, 7, 9 ou 11 ; et x égal à 1 ou 2.

Le premier monomère fluoré peut aussi être de formule : 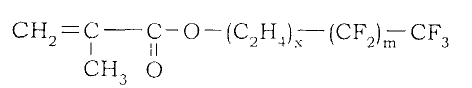 avec m allant de 4 à 16, de préférence égal à 5, 7, 9 ou 11; et x égal à 1 ou 2.

Le premier monomère fluoré peut encore être de formule : 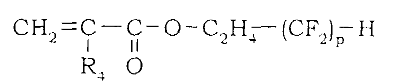 avec R4 représentant H ou CH₃, et p allant de 6 à 16, par exemple égal à 6 ou 8.

Le rapport en poids de premier monomère fluoré par rapport au second monomère non fluoré peut aller de 5/95 à 95/5, de préférence de 10/90 à 90/10, et par exemple de 12/88 à 50/50.

En particulier, les copolymères fluorés de l'invention peuvent être fabriqués comme décrit dans les documents FR-A-2175332, FR-A-2540131 et EP-A-206671. Ces copolymères sont par exemple ceux vendus par ATOCHEM sous la marque Foraperle ® 344. On peut aussi utiliser les Foraperle ® 145, 333, 390, 350 qui sont des monomères fluorés aminés.

Ces polymères fluorés se présentent sous forme de particules en dispersion dans l'eau, la dispersion contenant éventuellement un alcool et/ou une cétone en faible quantité, dispersion que l'on peut diluer en toute proportion dans l'eau.

La composition peut comprendre 1-60% en poids, de préférence 5-40% en poids de matière sèche de polymère fluoré filmogène. La teneur en matière sèche desdites dispersions aqueuses de polymère fluoré peut être de l'ordre de 5-60% en poids, et de préférence 15-30%. La taille des particules de polymère fluoré en dispersion aqueuse peut être comprise entre 10-500 nm, et est de préférence comprise entre 20 et 150 nm.

On peut associer au polymère fluoré, sous forme de dispersion aqueuse de polymère fluoré, une ou plusieurs autres matières filmogènes telles que les polymères hydrosolubles et/ou les polymères en dispersion aqueuse. Parmi les autres matières filmogènes utilisables dans la présente invention, on peut citer les polymères synthétiques, de type polycondensat ou de type radicalaire, les polymères d'origine naturelle, et leurs mélanges, sous forme hydrosoluble ou de préférence en dispersion aqueuse.

Parmi les polycondensats, on peut citer les polyuréthannes, les polyuréthannes/acryliques, les polyuréthannes/PVP, les polyester/polyuréthannes, les polyéther/polyuréthannes, les polyurées, les polyurée/polyuréthannes, les polyesters, les polyesters amides, les polyesters à chaîne grasse, les polyamides, les résines époxyesters, et leurs mélanges.

Les polymères de type radicalaire peuvent être notamment des polymères, ou des copolymères, acryliques et/ou vinyliques. On utilise de préférence des polymères radicalaires anioniques. Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.

Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.

On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle.

On peut également utiliser des copolymères acryliques/silicones, ou encore des copolymères nitrocellulose/acryliques.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides. La taille des particules de polymères non fluorés en dispersion aqueuse peut être comprise entre 10-500 nm, et est de préférence comprise entre 20 et 150 nm.

On peut utiliser de manière avantageuse des dispersions de polymères filmogènes non fluorés présentant une teneur en matière sèche de l'ordre de 5-60% en poids, et de préférence 30-40%. La composition peut comprendre 0-60% en poids, de préférence 5-40% en poids de matière sèche de polymère additionnel non fluoré.

De préférence, la composition comprend 10-40% en poids, préférentiellement 15-25% en poids, de matière sèche de polymères filmogènes totaux, à savoir fluorés et optionnellement non fluorés.

Les dispersions aqueuses de polymères filmogènes peuvent être préparées par l'homme du métier sur la base de ses connaissances générales.

Il est possible d'ajouter à la dispersion de polymère filmogène, fluoré ou non, un agent auxiliaire de filmification permettant d'obtenir les caractéristiques du film recherchées et/ou susceptible d'influencer ses caractéristiques physico-chimiques. Dans la présente description, on entend par 'dispersion de polymère filmogène', une dispersion susceptible de former un vrai film isolable, comprenant ou ne comprenant pas d'agent auxiliaire de filmification. Un tel agent auxiliaire de filmification peut être choisi parmi tous les composés connus de l'homme du métier comme étant susceptibles de remplir la fonction recherchée, et peut être notamment choisi parmi les agents plastifiants et/ou parmi les agents de coalescence. Cet agent auxiliaire peut être hydrosoluble ou insoluble dans l'eau et peut éventuellement se présenter sous forme de dispersion aqueuse.

En particulier, on peut citer, seuls ou en mélange, les plastifiants ou agents de coalescence usuels, tels que :
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther,
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ou en dispersion aqueuse, ayant une température de transition vitreuse faible, inférieure à 25°C, de préférence inférieure à 15°C.

La quantité d'agent auxiliaire de filmification peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

Le système polymérique selon l'invention comprend donc au moins un polymère fluoré filmogène, en dispersion aqueuse, éventuellement une ou plusieurs dispersions aqueuses de particules de polymère non fluoré filmogène, et éventuellement un ou plusieurs agents auxiliaires de filmification (agents de coalescence et/ou plastifiants).

Dans un mode de réalisation préféré, on choisit un système polymérique permettant l'obtention d'un film ayant au moins l'une des caractéristiques physico-chimiques suivantes :
- une élongation supérieure à environ 200%, de préférence supérieure à 300%, et/ou
- une dureté inférieure à environ 110, de préférence comprise entre 1 et 70, et préférentiellement entre 5 et 55, et/ou
- un module de Young inférieur à environ 200 MPa, de préférence inférieur à environ 100 MPa, et préférentiellement compris entre 5 et 80 MPa.
Les méthodes de mesure d'élongation, de dureté et de module de Young (module d'élasticité) sont décrites avant les exemples.

De plus, la température de transition vitreuse (Tg) du système polymérique selon l'invention est avantageusement inférieure ou égale à environ 10°C, de préférence inférieure ou égale à environ 0°C, préférentiellement encore inférieure ou égale à environ -10°C et de façon encore plus préférée inférieure ou égale à environ-20°C.

D'autre part, la température minimale de filmification (TMF) du système polymérique selon l'invention est avantageusement inférieure ou égale à environ 12°C, de préférence inférieure ou égale à environ 8°C, préférentiellement encore inférieure ou égale à 5°C et de façon encore plus préférée inférieure ou égale à 2°C.

L'application, notamment sur les lèvres, d'une composition comprenant un tel système polymérique laisse un film qui présente une très bonne tenue sur les lèvres. Le film suit bien le mouvement des lèvres sans se décoller ni craqueler.

Les compositions selon l'invention peuvent comprendre, en outre, au moins un agent épaississant en une proportion de 0,01-5%, de préférence 0,1-1%, en poids par rapport au poids total de la composition. Parmi les agents épaississants appropriés pour la formulation de ces compositions filmogènes aqueuses, on peut citer la cellulose et ses dérivés telles que la carboxyméthylcellulose et l'hydroxyéthylcellulose, les silicates, les argiles telles que la laponite, les polymères synthétiques tels que les polymères acryliques ou les polymères associatifs de type polyuréthanne ou hydroxyéthylcellulose modifiés par une chaîne hydrophobe, et les gommes naturelles telles que la gomme de carraghénane ou de xanthane.

Par ailleurs, les compositions de l'invention peuvent comprendre un agent tensioactif pour émulsionner les copolymères fluorés dans l'eau, et donc favoriser leur dispersion. Ledit agent tensioactif présente notamment de préférence une valeur de HLB (équilibre lipophile/hydrophile) ≥ 10. Il peut être constitué par n'importe quel agent hydrophile cosmétiquement acceptable, notamment par un composé non ionique polyéthoxylé de HLB = 14 éventuellement associé à un ammonium quaternaire à chaîne grasse. En général, on utilise 0-10% en poids d'agent tensioactif par rapport au poids total de la composition et de préférence 3-5% en poids.

La composition peut en outre comprendre au moins un colorant hydrosoluble et/ou au moins un pigment, utilisés de manière usuelle dans le domaine de la cosmétique et du maquillage. Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium. Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, leurs mélanges.

On peut également ajouter dans la composition selon l'invention tout additif connu tel que des agents d'étalement, des dispersants, des conservateurs, des agents antimousses, des agents mouillants, des filtres UV, des parfums, des charges, des actifs cosmétiques ou pharmaceutiques, des hydratants, des vitamines et leurs dérivés, des matières biologiques et leurs dérivés.

La composition peut également comprendre une dispersion aqueuse de cire, de préférence une microdispersion aqueuse de cire. Parmi les cires susceptibles d'être utilisées, on peut notamment citer la cire d'abeilles, la cire de lanoline, et les cires d'insectes de Chine, la cire de riz, la cire de Carnauba, la cire de Candellila, la cire d'Ouricurry, la cire d'Alfa, la cire de fibres de liège, la cire de canne à sucre, la cire du Japon et la cire de sumac, la cire de montan, les cires microcristallines, les paraffines et l'ozokérite, les cires de polyéthylène, les cires obtenues par la synthèse de Fisher-Tropsch et les copolymères cireux ainsi que leurs esters. On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée. On peut encore citer les cires de silicone.

Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Le pH de la composition finale obtenue est de préférence inférieur à 9. Cette composition doit bien entendu être apte à se déposer sur un support tel que la peau, les muqueuses et/ou les semi-muqueuses.

La composition selon l'invention peut se présenter sous forme fluide, gélifiée, semi-solide, pâte souple, voire solide telle que de stick ou bâton. Dans le cas d'une formule non solide, la composition selon l'invention peut présenter une viscosité allant de 0,05 Pa.s à 20 Pa.s, et notamment de 0,05 Pa.s à 10 Pa.s, mesurée à 25 °C à l'aide d'un appareil Brookfield, mobile 4 LVT.

Elle trouve en particulier une application en tant que produit de maquillage, notamment en tant que rouge à lèvres, fond de teint, fard à joues ou fard à paupières, d'eye-liner ou encore de maquillage du corps. On peut également envisager une application dans le domaine des compositions de soin, notamment soin des lèvres, des compositions solaires ou autobronzantes, des compositions dermatologiques ou encore des compositions pharmaceutiques à appliquer sur la peau, les semi-muqueuses et/ou les muqueuses.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz.

Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### C/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.

Le film déposé sur le support doit avoir une épaisseur d'environ 300 microns avant séchage. Après séchage pendant 7 jours à 21°C et sous une humidité relative de 50%, on obtient un film ayant une épaisseur d'environ 100 microns. Les échantillons mesurés ont une largeur de 5 mm et une épaisseur de 100 microns. La distance entre les mors est de 25 mm. La vitesse de traction est de 1000 mm par minute.

### Exemple 1

On prépare un rouge à lèvres ayant la composition suivante :
- dispersion aqueuse de polymère acrylique fluoré (FORAPERLE 333 de ATOCHEM) 22 g MA
- dispersion de cire fluorée (MICRODISPERSION 411 de MICROPOWDERS) 5 g MA
- pigment 2 g
- glycérine 1,25 g
- eau qsp 100 g
MA : matière active

La composition s'étale facilement sur les lèvres et laisse sur celles-ci un film présentant une bonne tenue au cours du temps et qui ne transfère pas, confortable, non collant, non gras, résistant à l'eau.

### Exemple 2

On prépare un rouge à lèvre ayant la composition suivante :
- dispersion aqueuse de polymère acrylique fluoré (1) 30 g MA
- glycérine 10 g
- épaississant 0,5 g
- pigment 2 g
- eau qsp 100 g
(1) copolymère méthacrylate de méthyle/acrylate de butyle/méthacrylate de perfluorooctyl/acide acrylique (50/38/10/2) - taille des particules 130 nm.
(1) copolymère méthacrylate de méthyle/acrylate de butyle/méthacrylate de perfluorooctyl/acide acrylique (50/38/10/2) - taille des particules 130 nm.

On obtient une composition facile à appliquer sur les lèvres ; le film obtenu ne transfère pas et résiste à l'eau.

### Exemple 3

On prépare un rouge à lèvre ayant la composition suivante :
- dispersion aqueuse de polymère acrylique fluoré (1) 17,5 g MA
- dispersion aqueuse de résine acrylique (NEOCRYL A-523 de ZENECA) 17,5 g MA
- glycérine 5 g
- épaississant 0,6 g
- pigment 2 g
- eau qsp 100 g

On obtient une composition qui s'étale facilement sur les lèvres et laisse sur celles-ci un film présentant une bonne tenue et qui ne transfère pas, brillant, résistant à l'eau.

## Revendications

1. Composition susceptible d'être appliquée sur la peau, les semi-muqueuses et/ou les muqueuses, comprenant un système polymérique qui comprend une dispersion aqueuse de particules de polymère fluoré filmogène, ladite composition étant à phase continue aqueuse.

2. Composition sans transfert susceptible d'être appliquée sur la peau, les semi-muqueuses et/ou les muqueuses, comprenant un système polymérique qui comprend une dispersion aqueuse de particules de polymère fluoré filmogène.

3. Composition selon la revendication 1 ou 2 dans laquelle le système polymérique est présent en une quantité suffisante pour permettre l'obtention d'un film capable de suivre le mouvement de la peau, des semi-muqueuses et/ou des muqueuses sur lequel il est appliqué.

4. Composition selon l'une des revendications précédentes, dans laquelle le système polymérique est présent en une quantité suffisante pour permettre l'obtention d'un film sans transfert, de longue tenue et/ou de bonne résistance à l'eau.

5. Composition selon l'une des revendications précédentes, dans laquelle le système polymérique est présent en une quantité suffisante pour permettre l'obtention d'un film brillant.

6. Composition selon l'une des revendications précédentes, dans laquelle le polymère filmogène fluoré est présent dans un milieu cosmétiquement ou dermatologiquement acceptable.

7. Composition selon l'une quelconque des revendications précédentes, caractérisée en ce que le polymère fluoré est un polymère à squelette hydrocarboné dont au moins une partie des hydrogènes est substituée par des atomes de fluor.

8. Composition selon l'une des revendications précédentes, caractérisée en ce que le polymère fluoré est un perfluoroalkyl(méth)acrylate.

9. Composition selon l'une des revendications précédentes, dans laquelle le polymère fluoré résulte de la copolymérisation d'au moins un premier monomère fluoré vinylique et d'au moins un second monomère de formule (A) : 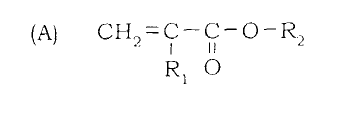 dans laquelle :
- R1 représente un atome d'hydrogène ou un radical alkyle en C1-C4 et
- R2 représente un radical alkyle en C1-C20, de préférence C2-C8, un radical hydroxycarboné en C2-C6, de préférence C2-C4, ou un radical -(CH₂)ₙ-NH-R3 avec R3 représentant un alkyle en C1-C6 ou un cycloalkyle et n étant un entier allant de 1 à 4.

10. Composition selon la revendication 9, dans laquelle le monomère de formule A est choisi parmi l'acide acrylique, l'acide méthacrylique, le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle, le (méth)acrylate d'hydroxyéthyle, le (méth)acrylate de 2-hydroxypropyle, leurs mélanges.

11. Composition selon l'une des revendications 9 à 10, dans laquelle le premier monomère fluoré est de formule (B) suivante : 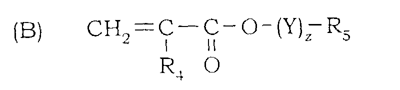 dans laquelle :
- R4 représente un atome d'hydrogène ou un radical alkyle en C1-C4,
- Y représente un radical alkylène en C1-C6 et
- z est égal à 0 ou 1,
- R5 représente un radical alkyle en C1-C20 dont tout ou partie des atomes d'hydrogène est remplacé par des atomes de fluor.

12. Composition selon la revendication 11, dans laquelle le premier monomère fluoré est choisi parmi les monomères de formule : 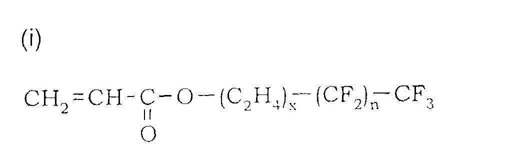 avec n allant de 4 à 16, de préférence égal à 5, 7, 9 ou 11 ; et x égal à 1 ou 2. 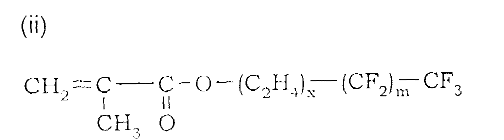 avec m allant de 4 à 16, de préférence égal à 5, 7, 9 ou 11 ; et x egal a 1 ou 2. 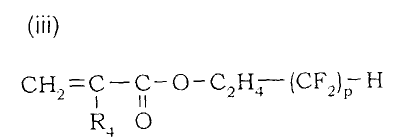 avec R4 représentant H ou CH₃, et p allant de 6 à 16, par exemple égal à 6 ou 8.

13. Composition selon l'une des revendications précédentes, comprenant 1-60% en poids, de préférence 5-40% en poids de matière sèche de polymère fluoré filmogène.

14. Composition selon l'une des revendications précédentes, dans laquelle la teneur en matière sèche des dispersions aqueuses de polymère fluoré est de l'ordre de 5-60% en poids, et de préférence 15-30%.

15. Composition selon l'une des revendications précédentes, dans laquelle la taille des particules de polymère fluoré en dispersion aqueuse est comprise entre 10-500 nm, de préférence entre 20 et 150 nm.

16. Composition selon l'une des revendications précédentes, comprenant en outre une ou plusieurs autres matières filmogènes telles que les polymères hydrosolubles et/ou les polymères en dispersion aqueuse.

17. Composition selon l'une des revendications précédentes, comprenant en outre un agent auxiliaire de filmification.

18. Composition selon l'une des revendications précédentes, comprenant en outre au moins un colorant hydrosoluble et/ou au moins un pigment.

19. Composition selon l'une des revendications précédentes, comprenant en outre une dispersion aqueuse de cire, de préférence une microdispersion aqueuse de cire.

20. Composition selon l'une des revendications précédentes, dans laquelle le système polymérique permet l'obtention d'un film ayant au moins l'une des caractéristiques physico-chimiques suivantes :
- une élongation supérieure à environ 200%, de préférence supérieure à 300%, et/ou
- une dureté inférieure à environ 110, de préférence comprise entre 1 et 70, et préférentiellement entre 5 et 55, et/ou
- un module de Young inférieur à environ 200 MPa, de préférence inférieur à environ 100 MPa, et préférentiellement compris entre 5 et 80 MPa.

21. Composition selon l'une des revendications précédentes, dans laquelle la température de transition vitreuse (Tg) du système polymérique est inférieure ou égale à environ 10°C, de préférence inférieure ou égale à environ 0°C, préférentiellement encore inférieure ou égale à environ -10°C et de façon encore plus préférée inférieure ou égale à environ -20°C.

22. Composition selon l'une des revendications précédentes, dans laquelle la température minimale de filmification (TMF) du système polymérique est inférieure ou égale à environ 12°C, de préférence inférieure ou égale à environ 8°C, préférentiellement encore inférieure ou égale à 5°C et de façon encore plus préférée inférieure ou égale à 2°C.

23. Composition selon l'une des revendications précédentes, se présentant sous la forme d'un produit de maquillage, notamment en tant que rouge à lèvres, fond de teint, fard à joues, fard à paupières, eye-liner, maquillage du corps ; d'une composition de soin, notamment soin des lèvres ; d'une composition solaire ou autobronzante ; d'une composition dermatologique ou pharmaceutique.

24. Composition de rouge à lèvres ou de soin des lèvres, caractérisée par le fait que la composition est conforme à l'une quelconque des revendications 1 à 23.

25. Composition de maquillage semi-permanent du corps, caractérisée en ce qu'elle est conforme à l'une quelconque des revendications 1 à 23.

26. Procédé de maquillage d'un support choisi parmi la peau, les semi-muqueuses et les muqueuses d'être humain, caractérisé par le fait que l'on applique sur ledit support, une composition selon l'une quelconque des revendications 1 à 23.

27. Procédé de traitement non thérapeutique d'un support choisi parmi la peau, les semi-muqueuses et les muqueuses d'être humain, caractérisé par le fait que l'on applique sur ledit support, une composition selon l'une des revendications 1 à 23.

28. Utilisation d'un système polymérique comprenant une dispersion aqueuse de particules de polymère fluoré filmogène, dans une composition à appliquer sur un support choisi parmi la peau, les semi-muqueuses et les muqueuses d'être humain, pour obtenir un film capable de suivre le mouvement dudit support.

29. Utilisation selon la revendication 28, pour diminuer le transfert et/ou la migration de ladite composition.

30. Utilisation selon l'une des revendications 28 à 29, pour obtenir un film sans transfert et/ou de longue tenue et/ou résistant à l'eau.

31. Utilisation selon l'une des revendications 28 à 30, pour obtenir un film brillant.

32. Utilisation selon l'une des revendications 28 à 30, pour obtenir un film souple et/ou élastique et/ou flexible sur la peau et/ou les lèvres qui ne se craquèle pas et/ou qui ne se décolle pas.
